Europäisches Patentamt

European Patent Office

Office européen des brevets

(19)

(11) Numéro de publication: **0 373 082**
**A1**

(12) # DEMANDE DE BREVET EUROPEEN

(21) Numéro de dépôt: **89420480.9**

(22) Date de dépôt: **05.12.89**

(51) Int. Cl.⁵: **C07C 59/185, C07C 51/00**

(30) Priorité: **09.12.88 FR 8816509**

(43) Date de publication de la demande:
**13.06.90 Bulletin 90/24**

(84) Etats contractants désignés:
**AT BE CH DE ES FR GB GR IT LI LU NL SE**

(71) Demandeur: **SOCIETE FRANCAISE D'ORGANO-SYNTHESE**
**La Defense 3 - Les Miroirs - 18, Avenue d'Alsace**
**F-92400 Courbevoie(FR)**

(72) Inventeur: **Capai, Bernard**
**28 rue de la Gare**
**F-95270 Viarmes(FR)**
Inventeur: **Lartigau, Guy**
**2 rue Alfred de Vigny**
**F-91600 Savigny sur Orge(FR)**

(74) Mandataire: **Vignally, Noel et al**
**Rhône-Poulenc Chimie Service Brevets**
**Chimie Centre de Recherches des Carrières**
**B.P. 62**
**F-69192 Saint-Fons Cédex(FR)**

(54) **Préparation d'acide lévulinique.**

(57) La présente invention concerne la préparation d'acide lévulinique à partir d'alcool furfurylique.

Plus précisément, il s'agit d'un procédé de préparation d'acide lévulinique, par chauffage d'alcool furfurylique en présence d'eau et d'un acide protonique fort, non oxydant dans les conditions opératoires, caractérisé en ce que l'alcool furfurylique est introduit progressivement dans un mélange comprenant l'eau, l'acide protonique fort et de l'acide lévulinique comme solvant de la réaction.

## PREPARATION D'ACIDE LEVULINIQUE

La présente invention concerne la préparation d'acide lévulinique à partir d'alcool furfurylique.

L'acide lévulinique ou acide oxo-4 pentanoïque sert d'intermédiaire dans la synthèse de produits pharmaceutiques ou d'autres composés chimiques.

Sa préparation se fait selon deux voies différentes.

On peut l'obtenir par chauffage de sucres tels que le glucose, le saccharose, les mélasses ou l'amidon dans l'acide chlorhydrique dilué. Cette préparation ne donne pas de rendements élevés et ne peut guère être utilisée industriellement.

On peut également préparer l'acide lévulinique par ouverture du cycle de l'alcool furfurylique dans l'eau et en présence d'une huile. Ainsi le brevet américain n° 3 752 849 décrit un tel procédé en utilisant une cétone hydrosoluble comme solvant de la réaction. Un tel procédé conduit à de bons rendements. Cependant la cétone utilisée comme solvant intervient elle-même dans des réactions secondaires d'aldolisation et de condensation. On obtient par ce procédé un acide lévulinique coloré même après distillation et dont le titre ne dépasse pas 98 %, en raison des grandes difficultés de séparation des impuretés qu'il contient.

La présente invention se propose de simplifier le procédé de préparation d'acide lévulinique à partir d'alcool furfurylique et de pallier les inconvénients du procédé antérieur, en permettant, par simple distillation, d'obtenir un acide lévulinique pratiquement incolore et de pureté supérieure à 98 %.

Plus précisément, il s'agit d'un procédé de préparation d'acide lévulinique, par chauffage d'alcool furfurylique en présence d'eau et d'un acide protonique fort, non oxydant dans les conditions opératoires, caractérisé en ce que l'alcool furfurylique est introduit progressivement dans un mélange comprenant l'eau, l'acide protonique fort et de l'acide lévulinique comme solvant de la réaction.

Dans le présent texte, l'acide protonique fort sera défini comme un acide protonique présentant une fonction d'acidité Ho inférieure ou égale à - 4.

Comme exemples non limitatifs de tels acides protoniques, on peut citer plus particulièrement les acides halohydriques tels que l'acide chlorhydrique, l'acide bromhydrique et l'acide iodhydrique ; l'acide sulfurique, l'acide pyrosulfurique, l'acide perchlorique, les acides phényl-sulfoniques, l'acide méthanesulfonique, l'acide trifluorométhanesulfonique, l'acide chlorosulfonique, l'acide fluorosulfonique.

Les acides halohydriques qui n'ont pas d'action oxydante sont préférés.

En pratique, on utilisera généralement l'acide chlorhydrique qui convient très bien et qui est bon marché.

La quantité d'eau mise en oeuvre peut varier dans de très larges limites.

Habituellement on utilise de 1 mole à 25 moles d'eau pour 1 mole d'alcool furfurylique et de préférence de 1,5 mole à 10 moles d'eau pour 1 mole d'alcool furfurylique.

La quantité d'acide lévulinique engagée comme solvant de la réaction peut également varier dans de larges limites.

On introduira en général une quantité d'acide lévulinique, représentant en poids par rapport au poids total d'alcool furfurylique mis en oeuvre dans la réaction de 30 % à 1000 % et de préférence de 50 % à 500 %.

La quantité d'acide protonique fort engagée est telle qu'elle représente de 1 % à 50 % par rapport au poids total d'eau mis en oeuvre et de préférence de 2 % à 20 %.

La température à laquelle est réalisé le procédé selon l'invention est de préférence égale ou supérieure à 60° C.

La limite supérieure est conditionnée essentiellement par la température d'ébullition des différents constituants du mélange réactionnel et de la pression sous laquelle on opère.

Sous pression atmosphérique, la limite supérieure de la température sera 100° C. Si l'on opère en enceinte fermée, sous la pression autogène des constituants du mélange réactionnel la limite supérieure sera de 250° C.

L'alcool furfurylique est introduit progressivement, par coulée, par injection ou par tout un autre moyen, dans le réacteur contenant l'acide lévulinique, l'eau et l'acide protonique fort.

La durée de cette introduction peut varier de quelques minutes à plusieurs dizaines d'heures, en fonction des rapports des réactifs et de l'agitation notamment.

Généralement la durée d'introduction est de 1 heure à 12 heures sans que ces valeurs indicatives aient un caractère critique.

Le traitement du mélange réactionnel final et la séparation de l'acide lévulinique est très simplifiée dans le présent procédé. Il suffit, après la fin de la réaction, d'éliminer par distillation sous pression réduite l'eau excédentaire et l'acide protonique fort, lorsque celui-ci a un point d'ébullition suffisamment bas, comme l'acide chlorhydrique, l'acide bromhydrique et l'acide iodhydrique.

Si l'acide protonique fort n'est pas volatil, il faudra tout d'abord le neutraliser, par exemple à l'aide d'un hydroxyde ou d'un carbonate de métal alcalin, puis séparer le sel formé par toute méthode connue.

L'acide lévulinique est dosé, par exemple par potentiomètrie dans le mélange brut.

La séparation de l'acide lévulinique des sous-produits, qui peuvent se former au cours de la réaction et qui sont notamment des composés de nature polymérique formés par condensation de l'alcool furfurylique sur lui-même, se fait par distillation sous pression réduite.

Le présent procédé permet d'obtenir un acide lévulinique de grande pureté et ne présentant pas de coloration ou une très légère coloration jaune très pâle. L'absence d'un tiers solvant évite la formation de divers sous-produits résultant de réactions secondaires faisant intervenir ledit tiers solvant. Ces sous-produits sont généralement très colorés, notamment ceux qui proviennent de l'utilisation d'une cétone comme solvant et sont en général très difficiles à séparer de l'acide lévulinique.

Les exemples qui suivent illustrent la présente invention.

Exemple 1

Dans un réacteur en verre de 6 litres, muni d'un agitateur, d'une ampoule de coulée et d'un thermomètre, on charge :
- 2550 g d'acide lévulinique (à 98 %)
- 990 g d'eau
- 150 g d'une solution aqueuse d'acide chlorhydrique à 37 % en poids.

On agite et on chauffe le mélange réactionnel à 90° C.

En maintenant la température à 90° C, on coule en 6 heures 2940 g (30 moles) d'alcool furfurylique.

Après la fin de coulée, on distille sous pression réduite l'eau excédentaire et l'acide chlorhydrique.

On obtient 5841 g d'une huile fluide, brune qui titre (par potentiométrie) 92,4 % en acide lévulinique (soit, après déduction de l'acide lévulinique engagé, un rendement en acide lévulinique formé, non isolé, de 83,3 % par rapport à l'alcool furfurylique engagé).

L'acide lévulinique brut est rectifié sous pression réduite (670 Pa) ; on obtient :
- température des vapeurs d'acide lévulinique sous 670 Pa : de 122° C à 124° C,
- couleur de l'acide lévulinique distillé (selon l'échelle de coloration GARDNER : 1 à 2, c'est-à-dire d'incolore à jaune très pâle),
- poids de l'acide lévulinique distillé : 5455 g
- titre de l'acide lévulinique distillé : 98,8 %
- rendement (après déduction de l'acide lévulinique engagé comme solvant) en acide lévulinique isolé par rapport à l'alcool furfurylique engagé : 83,0 %.

Essai comparatif A

Dans un réacteur en verre de 6 litres, muni d'un agitateur, d'une ampoule de coulée et d'un thermomètre, on charge :
- 2160 g de méthyl-éthyl-cétone
- 252 g d'eau
- 252 g d'une solution aqueuse d'acide chlorhydrique à 37 % en poids.

On agite et on chauffe le mélange réactionnel à reflux (80° C).

En maintenant la température à 80° C, on coule en 6 heures 1646 g (16,8 moles) d'alcool furfurylique.

Après la fin de coulée, on distille sous pression réduite la méthyl-éthyl-cétone, l'eau excédentaire et l'acide chlorhydrique.

On obtient 1812 g d'une huile fluide, brune qui titre (par potentiométrie) 82,0 % en acide lévulinique, (soit un rendement en acide lévulinique formé, non isolé, de 76,2 % par rapport à l'alcool furfurylique engagé).

L'acide lévulinique brut est rectifié sous pression réduite (670 Pa) ; on obtient :
- température des vapeurs d'acide lévulinique sous 670 Pa : de 123° C à 125° C,
- couleur de l'acide lévulinique distillé (selon l'échelle de coloration GARDNER : 3 à 4, c'est-à-dire jaune),
- poids de l'acide lévulinique distillé : 1530 g
- titre de l'acide lévulinique distillé : 97,0 %
- rendement en acide lévulinique isolé par rapport à l'alcool furfurylique engagé : 76,1 %.

Après bidistillation, le titre de l'acide lévulinique demeure à 97,0 %. L'acide lévulinique reste souillé par des produits d'aldolisation-crotonisation de la méthyl-éthyl-cétone (identification par chromatographie gaz-liquide et par infra-rouge).

Essai comparatif B

Dans un réacteur en verre de 6 litres, muni d'un agitateur, d'une ampoule de coulée et d'un thermomètre, on charge :
- 1950 g de méthyl-isobutyl-cétone
- 680 g d'eau
- 441 g d'une solution aqueuse d'acide chlorhydrique à 37 % en poids.

On agite et on chauffe le mélange réactionnel à 80° C.

En maintenant la température à 80° C, on coule en 4 heures une solution comprenant 294 g (3 moles) d'alcool furfurylique dans 600 g de méthyl-isobutyl-cétone.

Après la fin de coulée, on distille sous pression réduite la méthyl-isobutyl-cétone, l'eau excédentaire et l'acide chlorhydrique.

On obtient 328,5 g d'une huile fluide, brune.

L'acide lévulinique brut est rectifié sous pression réduite (670 Pa) ; on obtient :
- couleur de l'acide lévulinique distillé (selon l'échelle de coloration GARDNER : 3 à 4, c'est-à-dire jaune),
- poids de l'acide lévulinique distillé : 267 g
- titre de l'acide lévulinique distillé : 94,0 %
- rendement en acide lévulinique isolé par rapport à l'alcool furfurylique engagé : 72,1 %.

Après bidistillation, le titre de l'acide lévulinique demeure à 94,0 %. L'acide lévulinique reste souillé par des produits d'aldolisation-crotonisation de la méthyl-isobutyl-cétone (identification par chromatographie gaz-liquide et par infra-rouge).

Exemple 2

Dans un réacteur en verre de 1 litre, muni d'un agitateur, d'une ampoule de coulée et d'un thermomètre, on charge :
- 255 g d'acide lévulinique (à 98 %)
- 99 g d'eau
- 26,2 g d'une solution aqueuse d'acide bromhydrique à 47 % en poids.

On agite et on chauffe le mélange réactionnel à 90° C.

En maintenant la température à 90° C, on coule en 6 heures 294 g (3 moles) d'alcool furfurylique.

Après la fin de coulée, on distille sous pression réduite l'eau excédentaire et l'acide bromhydrique.

On obtient 580 g d'une huile fluide, brune qui titre (par potentiométrie) 88 % en acide lévulinique (soit, après déduction de l'acide lévulinique engagé, un rendement en acide lévulinique formé, non isolé, de 73,3 % par rapport à l'alcool furfurylique engagé).

**Revendications**

1. Procédé de préparation d'acide lévulinique, par chauffage d'alcool furfurylique en présence d'eau et d'un acide protonique fort, non oxydant dans les conditions opératoires, caractérisée en ce que l'alcool furfurylique est introduit progressivement dans un mélange comprenant l'eau, l'acide protonique fort et de l'acide lévulinique comme solvant de la réaction.

2. Procédé selon la revendication 1, caractérisé en ce que l'acide protonique fort utilisé est un acide protonique présentant une constante d'acidité Ho inférieure ou égale à - 4.

3. Procédé selon la revendication 1 ou 2, caractérisé en ce que l'acide protonique fort utilisé est choisi parmi l'acide chlorhydrique, l'acide bromhydrique, l'acide iodhydrique, l'acide sulfurique, l'acide pyrosulfurique, l'acide perchlorique, les acides phényl-sulfoniques, l'acide méthanesulfonique, l'acide trifluorométhanesulfonique, l'acide chlorosulfonique, l'acide fluorosulfonique.

4. Procédé selon l'une des revendications 1 à 3, caractérisé en ce que l'acide protonique fort utilisé est l'acide chlorhydrique, l'acide bromhydrique ou l'acide iodhydrique, et de préférence l'acide chlorhydrique.

5. Procédé selon l'une des revendications 1 à 4, caractérisé en ce que l'on utilise de 1 mole à 25 moles d'eau pour 1 mole d'alcool furfurylique et de préférence de 1,5 mole à 10 moles d'eau pour 1 mole d'alcool furfurylique.

6. Procédé selon l'une des revendications 1 à 5, caractérisé en ce que l'on utilise une quantité d'acide lévulinique, représentant en poids par rapport au poids total d'alcool furfurylique mis en oeuvre dans la réaction de 30 % à 1000 % et de préférence de 50 % à 500 %.

7. Procédé selon l'une des revendications 1 à 6, caractérisé en ce que la quantité d'acide protonique fort engagée est telle qu'elle représente de 1 % à 50 % par rapport au poids total d'eau mis en oeuvre et de préférence de 2 % à 20 %.

8. Procédé selon l'une des revendications 1 à 7, caractérisé en ce que la température à laquelle est réalisé le procédé selon l'invention est de préférence égale ou supérieure à 60° C.

9. Procédé selon l'une des revendications 1 à 8, caractérisé en ce que l'on opère sous pression atmosphérique à une température comprise entre 60° C et 100° C.

10. Procédé selon l'une des revendications 1 à 8, caractérisé en ce que l'on opère sous pression autogène des constituants du mélange réactionnel, à une température comprise entre 60° C et 250° C.

11. Procédé selon l'une des revendications 1 à 10, caractérisé en ce que l'alcool furfurylique est introduit progressivement en une durée de quelques minutes à plusieurs dizaines d'heures et de préférence de 1 heure à 12 heures.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | DE-C- 685 143 (HOLZHYDROLYSE AG) * Revendications 1-2; page 2, lignes 29-36 * | 1-4 | C 07 C 59/185 C 07 C 51/00 |
| Y | PATENT ABSTRACTS OF JAPAN, vol. 12, no. 132 (C-490)[2979], 22 avril 1988; & JP-A-62 252 742 (UBE IND. LTD) 04-11-1987 * Résumé * | 1-4 | |
| A | US-A-2 738 367 (REDMON) * Revendication 1 * | 1 | |

DOMAINES TECHNIQUES
RECHERCHES (Int. Cl.5)

C 07 C 59/00
C 07 C 51/00

Le présent rapport a été établi pour toutes les revendications

| Lieu de la recherche | Date d'achèvement de la recherche | Examinateur |
|---|---|---|
| LA HAYE | 22-02-1990 | KLAG M.J. |

EPO FORM 1503 03.82 (P0402)